# FASCICULE DE BREVET EUROPEEN

(11) **EP 1 166 119 B1**
(45) Date de publication et mention de la délivrance du brevet: **14.03.2007**
(21) Numéro de dépôt: 00917154.7
(22) Date de dépôt: 07.04.2000
(51) Int. Cl.: G01N 33/58, C12Q 1/42, C12Q 1/34, C12Q 1/68

(54) **METHODE HOMOGENE DE DETECTION METTANT EN JEU UN CRYPTATE DE TERRE RARE**
HOMOGENE NACHWEISMETHODE UNTER VERWENDUNG VON KRYPTATEN DER SELTENEN ERDEN
HOMOGENEOUS DETECTION AND/OR DETERMINATION METHOD FOR A CHEMICAL OR PHYSICO-CHEMICAL INTERACTION

(30) Priorité: 08.04.1999 FR 9904382
(43) Date de publication de la demande: 02.01.2002
(73) Titulaire: CIS BIO INTERNATIONAL, 91400 Saclay (FR)
(72) Inventeur: MATHIS, Gérard, F-30200 Bagnols sur Cèze (FR); BAZIN, Hervé, F-30400 Villeneuve Lès Avignon (FR)
(74) Mandataire: Gillard, Marie-Louise
(86) Numéro de dépôt international: PCT/FR2000/000887
(87) Numéro de publication internationale: WO 2000/062072

(56) Documents cités:
- EP-A- 0 321 353
- WO-A-91/08490
- WO-A-92/01224
- WO-A-98/15830
- FR-A- 2 769 315
- US-A- 5 827 653

## Description

L'invention concerne une méthode homogène de détection et/ou de détermination par fluorescence d'une interaction chimique ou physicochimique dans un milieu de mesure, dans laquelle on utilise un cryptate de terre rare comportant un substituant, caractérisée en ce qu'on mesure la variation d'au moins une caractéristique de fluorescence du cryptate de terre rare induite par la modification des propriétés physicochimiques du substituant résultant de ladite interaction.

En biologie, il est intéressant de pouvoir mesurer ou étudier l'activité de différentes enzymes ou une interaction entre des biomolécules, qui sont des évènements clefs dans la compréhension des phénomènes cellulaires et dans la recherche de nouvelles molécules ou principes actifs.

Des méthodes dites homogènes (permettant de mesurer l'activité ou la liaison sans isoler du milieu les biomolécules étudiées) sont particulièrement intéressantes car elles permettent une automatisation des processus d'étude ou de mesure.

Les méthodes utilisant la fluorescence sont particulièrement sensibles mais sont susceptibles d'être perturbées par les propriétés optiques du milieu.

Une méthode homogène utilisant les cryptates de terre rare est particulièrement intéressante mais nécessite l'utilisation de deux marqueurs (G. Mathis et al., Clin. Chem., 1993, 39, 1251).

Dans la demande WO91/08490, on utilise astucieusement les différentes activités des enzymes à étudier pour apporter une modification chimique à une molécule substrat qui est capable, ou devient capable, de former un complexe en solution avec une terre rare.

Cette méthode permet en principe de détecter en phase homogène plusieurs types d'activité enzymatiques. Dans la pratique, la formation in situ du complexe avec la terre rare (en présence du milieu biologique) est un handicap important car le milieu peut contenir des molécules qui vont entrer en compétition avec le substrat modifié pour former le complexe.

Cette technique est surtout utilisée dans des dosages hétérogènes où l'on mesure l'activité d'une enzyme utilisée comme traceur (Elisa), lors de l'étude des propriétés photophysiques de différents cryptates et en particulier de cryptates portant des substituants fixés sur les motifs moléculaires donneurs.

WO 99/18114 décrit des conjugués fluorescents de nucléosides ou de nucléotides comprenant un cryptate de terre rare et leur utilisation dans un procédé de mesure de l'acitvité enzymatique d'une enzyme impliquée dans un réaction de synthèse d'acide nucléique (pe. polymérase à ADN ou ARN, transcriptase, transférase, ligase) par mesure de la fluorescence émise (directe ou indirecte). La mesure de fluorescence indirecte nécessite le rajout d'un deuxième traceur (FRET).

On a maintenant trouvé qu'une interaction chimique ou physicochimique susceptible de modifier un substituant lié à un cryptate de terre rare pouvait entraîner des modifications dans l'environnement de coordination de l'ion de terre rare et modifier par conséquent les propriétés phytophysiques du cryptate.

L'objet de l'invention est donc d'utiliser ces modifications de l'environnement de coordination d'un ion de terre rare dans un cryptate pour détecter ou mesurer une activité enzymatique ou une interaction biologique en phase homogène.

La méthode ne nécessite qu'un seul traceur fluorescent, à savoir le cryptate de terre rare.

En particulier, ladite modification influe sur la sphère de solvation de l'ion terre rare.

Ladite caractéristique de fluorescence est par exemple choisie parmi la durée de vie d'émission, l'intensité de fluorescence, la répartition des raies dans le spectre de fluorescence et la polarisation.

La variation mesurée dans la méthode selon l'invention peut par exemple être une augmentation ou une diminution de la durée de vie d'émission ou de l'intensité de fluorescence, et notamment être due à un agent induisant une exaltation ou une extinction de fluorescence.

Selon un aspect avantageux de la méthode selon l'invention, ledit substituant est chargé négativement ou acquiert une charge négative au cours d'un processus physicochimique.

Par « substituant », on entend dans la présente description aussi bien un groupement chimique tel que par exemple un groupe phosphate, phosphonate, carboxylate ou un de leurs dérivés, par exemple un dérivé ester ou amide, qu'une molécule chimique ou biologique comportant un tel groupe, telle que par exemple un acide nucléique, un oligonucléotide ou un oligonucléotide constitué de ou comprenant des analogues de nucléotides ; un nucléotide ou un nucléoside et leurs analogues.

L'interaction que l'on souhaite détecter peut par exemple résulter d'une réaction enzymatique, mettant en jeu notamment des enzymes telles que phosphatase, phosphodiestérase, estérase, exonucléase, déoxyribunocléase, ribonucléase, ligase, kinase ou polymérase.

Ladite interaction peut également, selon un autre aspect de l'invention, consister en une réaction d'hybridation ou de dénaturation d'acides nucléiques, d'oligonucléotides, ou d'oligonucléotides constitués de ou comprenant des analogues de nucléotides ou en une réaction entre lesdits acides nucléiques ou oligonucléotides avec des protéines ayant une affinité pour ceux-ci.

On entend par « analogue » de nucléotides ou d'oligonucléotides des molécules différentes des molécules naturelles correspondantes par modification de leur structure, tels que les isomères optiques (par exemple configuration α de la base par rapport au sucre), les isomères de position (par exemple enchaînements 2'-5' des ponts phosphate), ou par modification des substituants de la base (par exemple diaminopurine) ou du sucre (par exemple 3'-deoxyribose).

Ces oligonucléotides peuvent par exemple être constituées d'un enchaînement d'unités ribonucléotides ou désoxyribonucléotides ou d'unités analogues de nucléotides modifiées sur le sucre ou sur la base et liées entre elles par des liaisons intemucléotidiques naturelles de type phosphodiester, une partie des liaisons intemucléotidiques étant éventuellement remplacée par des liaisons phosphonate, phosphoramide ou phosphorothioate. Ces différentes familles d'oligonucléotides sont décrites dans Goodchild, *Bioconjugate Chemistry,* 1(3), May/June 1990, 77-99.

Ces oligonucléotides peuvent également être constitués d'un enchaînement comprenant à la fois des unités ribonucléotides ou désoxyribonucléotides liées entre elles par des liaisons de type phosphodiester et des unités analogues de nucléosides liées entre elles par des liaisons amides, communément dénommés « PNA » (en anglais « peptide nucleic acid »), tel que décrit dans M. Egholm et al., J. Am. Chem. Soc., 1992. 114, 1895-1897 ; de tels composés sont par exemple décrits dans R. Vinayak et al., Nucleoside & Nucleotide, 1997, 16 (7-9), 1653-1656.

La méthode selon l'invention permet notamment d'étudier, en temps réel, les aspects cinétiques d'une interaction entre molécules, par exemple l'hybridation, le fait d'utiliser un seul marqueur fluorescent en simplifiant considérablement la mise en oeuvre.

Dans un aspect avantageux de l'invention, le milieu de mesure est un milieu biologique, en particulier un milieu sérique.

Selon un aspect de l'invention, le cryptate de terre rare mis en oeuvre dans la méthode selon l'invention peut être lié au substituant, de manière covalente, soit directement, soit par l'intermédiaire d'un bras d'espacement.

Lorsque ledit substituant est un oligonucléotide, la liaison directe peut être réalisée sur un groupe fonctionnel préalablement introduit ou généré sur un ou plusieurs atomes d'une base ou d'une unité pentofuranose de l'oligonucléotide.

Dans la présente description, on désigne par groupe fonctionnel toute fonction portée par la partie nucléotidique ou introduite sur cette partie par toute méthode connue par l'homme du métier et capable de se lier par liaison covalente, directement ou après activation avec une fonction présente sur le cryptate ou sur le bras espaceur porté par le cryptate. De tels groupes fonctionnels sont notamment les fonctions NH₂, COOH, CHO, OH ou SH ainsi que les fonctions capables de donner des liaisons covalentes par substitution (halogénures, sulfonates, époxyde) ou par addition (double liaisons type maléïmide). Ces fonctions sont généralement portées par une chaîne hydrocarbonée elle-même reliée à la partie nucléotidique.

Des méthodes d'introduction de ces groupes fonctionnels sont notamment décrites dans C. Kessler, Nonisotopic probing, Blotting and Sequencing, 2^{nd} edition, L.J. Kricka (1995). Ed. Academic press Ltd., Londres, p. 66-72.Selon un aspect préféré de l'invention, le cryptate de terre rare est lié à l'oligonucléotide par l'intermédiaire d'un bras d'espacement . On entend par « bras d'espacement tout moyen permettant de lier de façon covalente l'oligonucléotide avec le cryptate au niveau d'un phosphate terminal, d'un atome d'une base purique ou pyrimidique ou d'un atome du sucre.

Dans un aspect avantageux, ledit bras d'espacement est constitué par un radical organique bivalent, choisi parmi les groupes alkylène linéaires ou ramifiés en C₁-C₂₀, contenant éventuellement une ou plusieurs doubles liaisons ou triples liaisons et/ou contenant éventuellement un ou plusieurs hétéroatomes, tels que l'oxygène, l'azote, le soufre, le phosphore ou un ou plusieurs groupe(s) carbamoyle ou carboxamido ; les groupes cycloalkylène en C₅-C₈ et les groupes arylène en C₆-C₁₄, lesdits groupes alkylène, cycloalkylène ou arylène étant éventuellement substitués par des groupes alkyle, aryle ou sulfonate.

En particulier, le bras d'espacement est choisi parmi les groupes de formules : 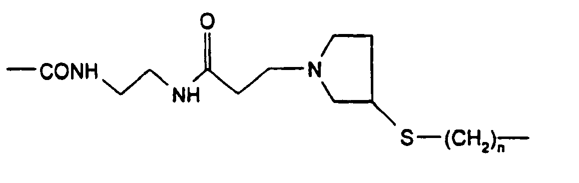 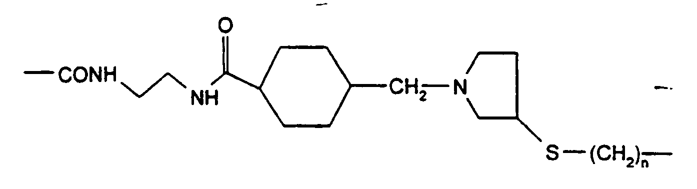 dans lesquelles n = 2 à 6, et -CONH-(CH₂)₆-,
la liaison via le groupe -CONH ayant lieu au niveau du cryptate.

Le cryptate de terre rare mis en oeuvre dans la mathode selon l'invention est constitué d'au moins un sel de terre rare complexé par un composé macropolycyclique de formule 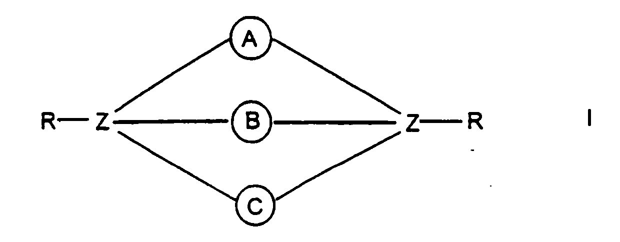 dans laquelle Z est un atome ayant 3 ou 4 valences, R est rien ou représente l'hydrogène, le groupe hydroxy, un groupe amino ou un radical hydrocarboné, les radicaux bivalents Ⓐ, Ⓑ et Ⓒ, sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ, Ⓑ et Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

En particulier, ledit cryptate de terre rare répond à la formule (I) dans laquelle le motif moléculaire est choisi parmi la phénanthroline, l'anthracène, le benzène, le naphtalène, les bi- et ter-phényle, l'azobenzène, l'azopyridine, la pyridine, les bipyridines, les bisquinoléines et les composés de formules ci-après :

- C₂H₄ - X₁ - C₆H₄ - X₂ - C₂H₄ -

- C₂H₄ - X₁ - CH₂ - C₆H₄ - CH₂ - X₂ - C₂H₄ -

X₁ et X₂ pouvant être identiques ou différents désignent l'oxygène, l'azote ou le soufre, 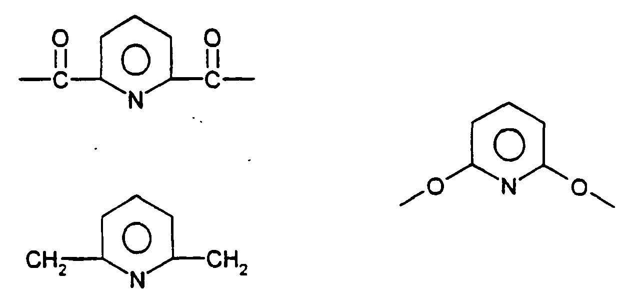 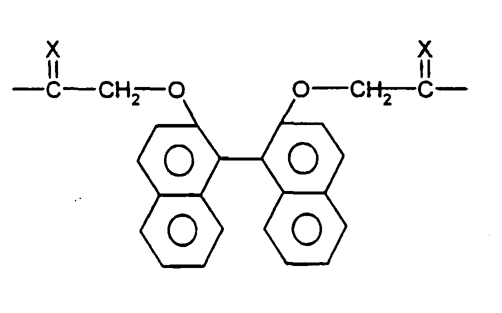 X étant l'oxygène ou l'hydrogène.

Avantageusement, ledit cryptate de terre rare est constitué d'un sel de terre rare complexé par l'un des composés macrocycliques ci-après :
(22)phénanthroline ; (22)phénanthroline amide ; (22)anthracène ; (22)anthracène amide ; (22)bi-isoquinoléine ; (22)biphényl-bis-pyridine ; (22)bipyridine ; (22)bi-pyridine amide ; les macropolycycles tris-bipyridine, tris-phénanthroline, phénanthroline-bis-bipyridine, bi-isoquinoléine-bis-bipyridine, bis-bipyridine diphénylbipyridine.

De tels composés sont par exemple décrits dans le brevet EP 180 492.

On peut également utiliser des composés macropolycycliques cryptates complexant des ions de terre rare dans lesquels le motif moléculaire est choisi parmi les bipyrazines, les bipyrimidines et les hétérocycles azotés comportant des groupes N-oxydes.

Des composés macropolycycliques à unités bipyrazines sont décrits dans F. Bodar-Houillon et al., New J. Chem., 1996, 20, 1041-1045.

Des composés macropolycycliques à unités bipyrimidines sont décrits dans J. M. Lehn et al., Helv. Chim. Acta, 1992, 75, 1221.

Des composés macropolycycliques comprenant des hétérocycles azotés comportant des groupes N-oxydes sont décrits dans J.M. Lehn et al., Helv. Chim. Acta, 1991, 74, 572.

Selon un autre aspect avantageux, ledit cryptate de terre rare est constitué d'au moins un sel de terre rare complexé par un composé macropolycyclique répondant à l'une des formules II ou III ci-après : 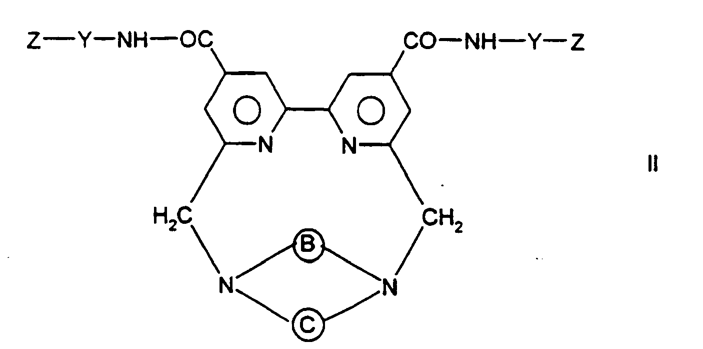 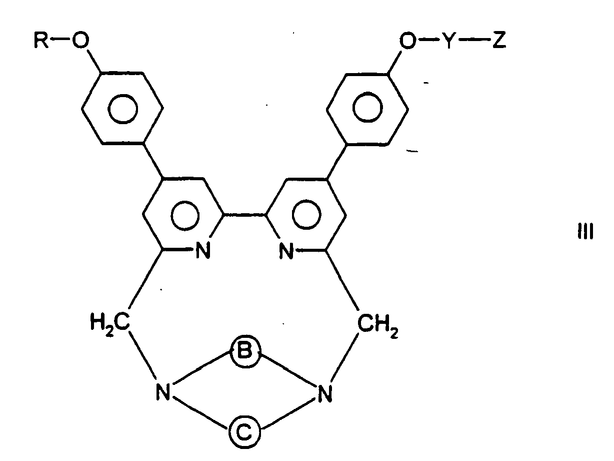 dans lesquels:
- le cycle de formule 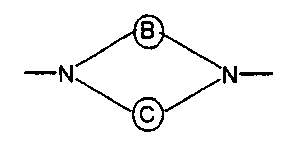 est l'un des cycles suivants :
   1) 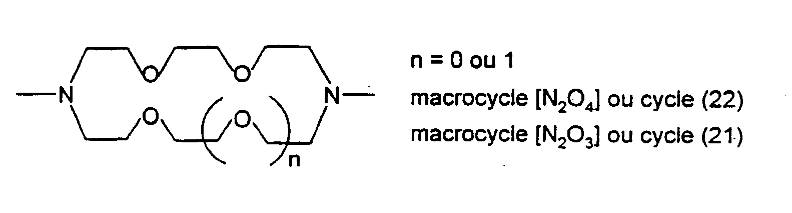
   2) 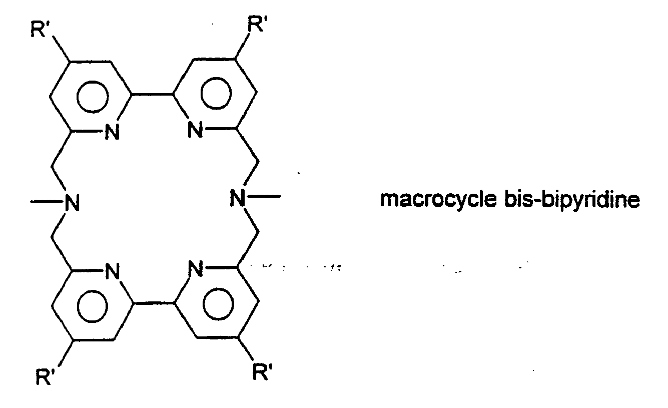
- Y est un groupe ou un bras d'espacement qui est constitué par un radical organique bivalent, choisi parmi les groupes alkylène linéaires ou ramifiés en C₁ à C₂₀ contenant éventuellement une ou plusieurs doubles liaisons et/ou éventuellement contenant un ou plusieurs hétéroatomes tels que l'oxygène, l'azote, le soufre, le phosphore ou un ou plusieurs groupe(s) carbamoyle ou carboxamido ; parmi les groupes cycloalkylène en C₅ à C₈ ou parmi les groupes arylène en C₆ à C₁₄, lesdits groupes alkylène, cycloalkylène ou arylène étant éventuellement substitués par des groupes alkyle, aryle ou sulfonate ;
- Z est un groupe fonctionnel susceptible de se lier de façon covalente avec une substance biologique ;
- R' est l'hydrogène ou un groupe -COOR" dans lequel R" est un groupe alkyle en C₁ à C₁₀ et représente de préférence le groupe méthyle, éthyle ou tertiobutyle ou bien R' est un groupe -CO-NH-Y-Z.

Selon un aspect avantageux, le cryptate de terre rare utilisé selon l'invention est un cryptate d'europium, de terbium, de samarium, de dysprosium ou de neodymium, le cryptate d'europium étant préféré.

Dans un aspect avantageux, ledit cryptate de terre rare est le cryptate d'europium Eu trisbipyridine ou Eu [bis-diéthoxybipyridine.bipyridine].

L'invention est illustrée par les exemples ci-après, dans lesquels on utilisera les abréviations suivantes :
ACN : acétonitrile
BSA : sérum albumine bovine
NHS/DCC : N-hydroxysuccinimide/dicyclohexylcarbodiimide
TEAB : hydrogénocarbonate de triéthylammonnium
TEA Ac : Acétate de triéthylammonium contenant 10 % d'acétonitrile.
TFA : acide trifluoroacétique
UF : unité arbitraire de fluorescence (dépend de l'appareil utilisé pour la mesure)

### Exemple 1 : Modification des propriétés photophysiques d'un conjugué cryptate-triphosphate et mesure de la durée de vie de fluorescence:

### 1°) Préparation d'un conjugué cryptate-triphosphate

On utilise un conjugué constitué d'une unité [Eu3+ (Trisbipyridine)] fonctionnalisée en position 4 d'une bipyridine et couplé par l'intermédiaire d'un bras espaceur à la position 5 d'une unité 2'-déoxynucléoside-5'-triphosphate ce conjugué sera dénommé K-11-dUTP. Le composé K-11-dUTP est préparé selon la procédure suivante :
Dans ce conjugué le, chiffre 11 indique le nombre total d'atomes du bras espaceur et du groupement fonctionnel qui relie la structure cryptate au nucléotide (ici, la liaison se fait en position 5 de la pyrimidine).

Le nucléoside-triphosphate utilisé est le 5-[N-(6-aminocaproyl)-3-aminoallyl]-2'-désoxyuridine-5'-triphosphate] (AH-dUTP) préparé par réaction du trifluoroacétamido-caproate de N-hydroxysuccinimide (M.S Urdea et al., Nucleic acids Res., 1988,.4937) sur le 5-(3-aminoallyl)-2'-désoxyuridine-5'-triphosphate préparé suivant un procédé de la littérature (Langer et al. Proc. Natl. Acad. Sci. USA (1981),78,6633-6637), suivi d'une déprotection ammoniacale (NH₄OH aqueuse 3 %, 45 min à 60°C). Le composé est purifié sur DEAE-Sepharose ^{®} (Pharmacia) dans un gradient linéaire d'hydrogénocarbonate de triéthylammonium (0,1 M à 0,3 M).

On dilue 68 µl d'une solution d'AH-dUTP à 6 µmole/ml (soit 0,4 µmole) à 250 µl d'hydrogénocarbonate de triéthylammonium (TEAB) 0,1 M pH 7,8 et on ajoute 320 µl d'une solution de cryptate de N-hydroxysuccinimide (4 mg/ml dans l'acétonitrile) préparée comme suit. Le cryptate d'Europium [(bis-bpy)-(bpy-diester)] décrit dans l'exemple 4, section A, de la demande EP 0 321 353 est hydrolysé par NaOH et le cryptate diacide obtenu est purifié sur colonne RP-18 HPLC (gradient d'acétonitrile dans de l'acide trifluoroacétique à 1% dans l'eau). Le cryptate d'Europium [(bis-bpy)-(bpy-diacide)] ainsi obtenu (4mg) est dissout dans 0,5 ml d'acétonitrile anhydre et on ajoute 1mg de N-hydroxysuccinimide, puis une solution de 1,9 mg de dicyclohexylcarbodiimide dissout dans 0,5 ml d'acétonitrile. Après 16 h de réaction, le précipité de dicyclohexylurée est filtré et la solution de cryptate de N-hydroxysuccinimide est utilisée directement pour le couplage.

Après 45 min sous agitation, on ajoute 15 µl de TEAB 1M pH 8,5 puis on injecte le mélange sur une colonne Superdex 30 ^{®} HR 10/30 (Pharmacia) en éluant par du TEAB 0,05M pH 7 contenant 10 % d'acétonitrile (débit 1 ml/min).

On collecte le composé de Rt ≅16,4 mn, puis cette fraction dénommée fraction 1 est concentrée sous vide (speed-vac) jusqu'à un volume de 350 µl et contient 8 UA₃₀₄ nm. En estimant un ε₃₀₄ ≅ 35000, on estime que la concentration en K-dUTP est d'environ 0,72 mM.

Une aliquote (90 µl) de cette fraction 1 est injectée sur la même colonne éluée par un tampon triéthylammonium acétate 25 mM pH 7. contenant 5 % d'acétonitrile. La fraction correspondant au seul pic du chromatogramme est collectée (16 min < Rt < 19 min) et concentrée sous vide (speed-vac). On obtient 150 µl d'une solution de K-dUTP dénommée fraction 2 contenant 1,95 UA₃₀₄ nm.

Le composé est analysé en spectrométrie de masse (électrospray mode positif) :
(M-2H)⁺ = 1431
(M-2H+CH₃COOH)⁺ = 1491.

Le spectre UV dans l'eau présente un maximum à 241 nm caractéristique de la partie nucléosidique de la molécule (λmax = 289 nm, ε = 7100, λmax = 240 nm ε = 10700) et un maximum à 304 nm proche du λmax de 305 nm (ε = 30000) caractéristique du cryptate d'Europium. On observe un rapport A₃₀₄/A₂₄₁ ≅ 0,83 compatible avec la structure proposée.

### 2°) Mesure de la cinétique d'hydrolyse d'un cryptate-triphosphate [trisbipyridine (Eu³⁺)-11-dUTP] par la phosphatase alcaline par mesure de durée de vie :

On introduit dans la cuve d'un spectrofluorimètre LS50 (Perkin-Elmer) 600 µl d'une solution de K-dUTP à une concentration de 1,6.10⁻⁶ M dans un tampon Tris 0,1 M pH 9,0 contenant 0,1M NaCl et 15mM MgCl₂.

On mesure l'intensité de fluorescence à 620 nm ainsi que la durée de vie de fluorescence [réglage du spectrofluorimètre : λexcitation=305 nm, λémission= 550 à 750 nm, mode phosphorescence 0,1 ms à 0,4 ms fente excitation=10 nm, fente émission=10 nm] qui donne la valeur initiale (t = 0).

On ajoute 2 µl d'une solution diluée au 1/100 ^{éme} de phosphatase alcaline (Boehringer) dans du tampon Tris 0,1M pH 9,0, ce qui correspond à 2 unités enzymatiques. Après des temps de 15 min, 30 min, 60 min et 80 min on mesure dans les mêmes conditions que ci-dessus l'intensité de fluorescence à 620 nm (spectre de fluorescence) ainsi que la durée de vie de l'Europium pour les temps 30 min, 60 min et 80 min.

Les valeurs observées sont rapportées dans le tableau 1 suivant.

**TABLEAU 1**

| temps (min) | émission à 620nm (UF) | durée de vie (ms) |
|---|---|---|
| 0 | 95 | 0,611 |
| 15 | 64 | nd |
| 30 | 47,5 | 0,568 |
| 60 | 29,8 | 0,521 |
| 80 | 24,5 | 0,51 |

Des mesures de fluorescences et de durées de vie dans des conditions semblables sont réalisées avec une solution de K-11-dUTP à une concentration de 3,7.10⁻⁶M dans le tampon ci-dessus puis on ajoute 1 µl de phosphatase alcaline diluée au 1/10 ème dans tampon Tris 0,1M pH 9,0, ce qui correspond à 10 unités enzymatiques.

Avant l'ajout de l'enzyme, on observe une durée de vie de 0,61 ms ; après l'ajout de l'enzyme, on observe que la durée de vie diminue très rapidement et se stabilise pour donner une valeur finale de 0,41 ms.

A titre de référence, on mesure le spectre et la durée de vie d'un cryptate [Eu3+ (tris-bipyridine) fonctionnalisé simplement par un bras espaceur, dénommé ci-après KNH2. On observe que la durée de vie du KNH2 dans le tampon Tris 0,1 M pH 9,0 est de 0,39 ms.

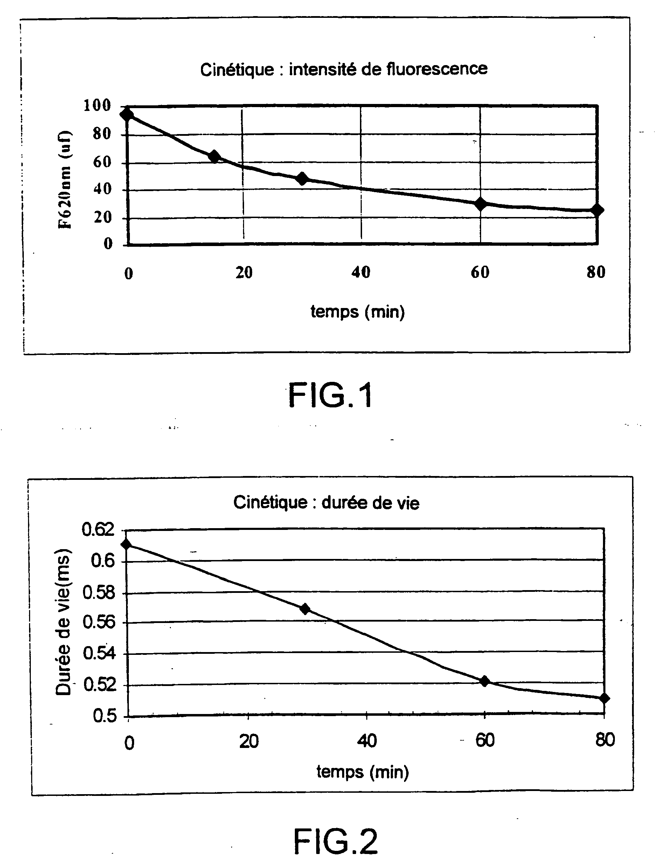

Les graphes des cinétiques de l'intensité de fluorescence et de la durée de vie sont représentés respectivement sur les figures 1 et 2.

On observe donc que l'hydrolyse enzymatique de la chaine triphosphate pour donner du phosphore inorganique s'accompagne d'une diminution de l'intensité de fluorescence et d'une diminution de la durée de vie. La cinétique de décroissance de la durée de vie (qui est indépendante de la concentration en composé fluorescent) est fonction de l'activité enzymatique ; elle est plus rapide si on augmente le nombre d'unités enzymatiques introduites.

La valeur finale de la durée de vie t = 0,41 ms après hydrolyse du K-dUTP tend vers la valeur de 0,39 ms observée pour le cryptate non conjugué.

L'hydrolyse des groupes phosphate change donc l'environnement du cryptate d'Europium et modifie ses propriétés photophysiques.

### Exemple 2 : Modification des propriétés photophysiques d'un conjugué cryptate-triphosphate et mesure de l'intensité de fluorescence en temps résolu

On prépare une solution de K-11-dUTP préparée comme indiqué dans l'exemple 1 à une concentration 3.10⁻⁶ M dans un tampon Tris 0,1 M pH 9,0 contenant 0,1M NaCl et 15mM MgCl₂. Parallèlement, on prépare une solution de KNH2 à une concentration de 3.10⁻⁶ M dans le même tampon.

Chaque solution est diluée au 1/500 ème M dans le même tampon et on dépose 200 µl de la solution diluée de K-11-dUTP dans 2 rangées de 10 puits d'une microplaque à fond noir. De la même façon on dépose 200 µl de la solution diluée de KNH2 dans 2 autres rangées de 10 puits de la microplaque.

On fait une lecture de la fluorescence à 620 nm (en utilisant une fenêtre de 50 à 400 µs) sur les différents puits grâce à un fluorimètre en temps résolu (Discovery) pour avoir les valeurs de fluorescence à t₀ = 0.

On ajoute 2 µl de tampon dans chacun des puits d'une rangée contenant le K-11-dUTP (dénommée KTP-T) et d'une rangée contant le KNH2 (dénommée K-T), on ajoute 2 µl d'une solution de phosphatase alcaline diluée au 1/100 ème (voir exemple 1) dans chacun des puits de l'autre rangée contenant le K-dUTP (dénommée KTP-E) et de l'autre rangée contant le KNH2 (dénommée K-E). On fait les moyennes des valeurs de fluorescence de chaque rangée.

On effectue les mesures à 620 nm au bout de 10 min, 35 min et 70 min. On mesure le rapport (Ft/F₀) de la fluorescence (Ft) au temps t sur la fluorescence (F₀) à t₀.

Les résultats sont rapportés dans le tableau 2 ci-dessous.

**TABLEAU 2**

| temps (min) | KTP-T | T (Ft/F₀) | KTP-E | R (Ft/F₀) |
|---|---|---|---|---|
| 0 | 174970 | 1 | 190100 | 1 |
| 10 | 193500 | 1,11 | 141900 | 0,75 |
| 35 | 198000 | 1,13 | 68000 | 0,36 |
| 70 | 195000 | 1,11 | 41500 | 0,22 |

Les résultats montrent qu'en présence de l'enzyme, l'intensité de la fluorescence à 620nm décroît au cours du temps. Cette évolution s'effectue à une vitesse comparable à ce qu'on peut attendre pour l'hydrolyse enzymatique des groupes phosphates d'un nucléoside triphosphate dans les conditions expérimentales choisies.

Par ailleurs, on observe que la fluorescence dans les puits KNH2 avec enzyme (K-E) est à la même intensité que dans les puits KNH2 témoins (K-T) sans enzyme et que dans les deux cas la fluorescence ne varie très peu au cours du temps, ce qui montre que le cryptate par lui-même n'est pas modifié par la présence de l'enzyme.

Ces résultats confirment que l'hydrolyse de la chaîne phosphate du K-11-dUTP provoque une modification des propriétés photophysiques du cryptate de terre rare.

### Exemple 3 : Préparation du cryptate [trisbipyridine(Eu3+)-aminohexylphosphate

### 1°) Préparation d'un conjugué cryptate [trisbipyridine(Eu3+)-aminohexylphosphate (KAH-PO4):

On dissout 2mg (10µmol) d'aminohexylephosphate (Sigma) dans 100 µl d'hydrogénocarbonate de triéthylammonium (TEAB) 0,1 M pH 7,8 et on ajoute 100 µl d'une solution de cryptate [TBP-(Eu3+)] activé (4 mg/ml dans l'acétonitrile) soit 0,26 µmol. Le cryptate [TBP-(Eu3+)] activé (NHS /DCC) dans l'acétonitrile comme décrit dans l'exemple 1, 1°) ci-dessus est préparée extemporanément à partir de cryptate d'Europium [(bis-bipy)-(bipy-diacide)] lui-même obtenu à partir du cryptate d'Europium [(bis-bipy)-(bipy-dimethylester)] décrit dans l'exemple 4, section A, de la demande EP 0 321 353.

Après 15 min sous agitation on concentre au demi, puis injecte le mélange sur une colonne Superdex peptide HR 10/30 (Pharmacia) en éluant par du TEAAc 25mM pH9 contenant 10% d'acétonitrile (débit 1ml/min).

On collecte le composé de Rt ≅19 min ; cette fraction est concentrée sous vide (speed-vac) jusqu'à un volume de 300 µl environ. Cette solution de KAH-PO4 purifié contient 0,2 µmole de cryptate et servira aux mesures photophysiques. Cette fraction est analysée sur la même colonne Superdex peptide éluée par un tampon triéthylammonium acétate 25 mM pH 7 contenant 10% d'acétonitrile. On observe un seul pic de Rt ≅ 20 min . La co-injection avec le cryptate d'Europium [(bis-bipy)-(bipy-diacide)] montre que ce dernier migre avec un Rt ≅ 23,7 min. De même on analyse le KAH-PO4 par RP18-HPLC (Merck) dans un gradient d'acétonitrile dans une solution aqueuse de TFA à 1% (ACN 15% isocratique pendant 5mn, puis gradient linéaire de 15 à 100 % d'ACN en 30 min). On observe un pic à Rt ≅ 16,6 min différent du cryptate d'Europium [(bis-bipy)-(bipy-diacide)] qui migre avec un Rt ≅15,9 min.

### 2°) Propriétés photophysiques du conjugué KAH-PO4:

A partir de la solution aqueuse du KAH-PO4 préparé comme ci-dessus, on prépare une solution diluée à 1,5.10⁻⁵ M de KAH-PO4 dans un tampon Tris 0,1 M pH 9,0 contenant 0.1M NaCl et 15mM MgCl₂.

On mesure l'intensité de fluorescence à 620nm ainsi que la durée de vie de fluorescence [réglage du spectrofluorimètre : λexcitation=305 nm, λémission= 550 à 750 nm, mode phosphorescence 0,1 ms à 0,4 ms fente excitation=10 nm, fente émission=10 nm] qui donne la valeur initiale ( t = 0).

On observe une durée de vie t = 0,637 ms.

On note que dans les mêmes conditions le cryptate d'Europium [(bis-bipy)-(bipy-diacide)] présente une durée de vie t = 0,21 ms.

### 3°) Cinétique de coupure des phosphates :

On utilise la solution de KAH-PO4 dans le tampon Tris décrite ci-dessus et on ajoute alors 2µl d'une solution diluée au 1/100ème de phosphatase alcaline (Boehringer) dans du tampon Tris 0,1M pH 9,0 ce qui correspond à 2 unités enzymatiques.

On effectue les mesures d'intensité de fluorescence et de durée de vie aux temps indiqués dans le tableau 3 ci-dessous :

**TABLEAU 3**

| temps (mn) | Intensité 620nm | Durée de vie (ms) |
|---|---|---|
| 0 | 130 | 0,633 |
| 5 | 86 | 0,597 |
| 10 | 74 | 0,578 |
| 15 | 64 | 0,566 |
| 20 | 55 | 0,555 |
| 30 | 48 | 0, 529 |
| 46 | 41 | 0,514 |
| 60 | 37 | 0,504 |
| 75 | 34 | 0,495 |

On laisse ensuite le mélange réactionnel 16h à 20°C pour que la réaction enzymatique soit totale. On observe alors une durée de vie t = 0,45 ms.

On observe une modification des propriétés photophysiques au cours de l'hydrolyse des fonctions phosphate. Au cours de la réaction, la durée de vie tend à se rapprocher de la valeur de durée de vie (t = 0,39 ms)observée pour KNH2 dans le même milieu.

### Exemple 4 : Hydrolyse d'un conjugué cryptate [trisbipyridine(Eu3+)]-oligonucléotide par phosphatase / phosphodiestérase]

### 1°) Marquage d'un oligodésoxynucléotide par un cryptate :

Un oligodésoxynucléotide (ODN)de ^{5'}d(T₁₀ G₄ C^{AH} G)₃ modifié près de son extrémité 3' par un bras aminohexyl (AH) est synthétisé sur support solide par la méthode dénommée « phosphite-phosphoramidite » en utilisant un synthétiseur d'ADN (Applied Biosystems type 392) selon le protocole du fabriquant. Un nucléotide modifié est introduit par couplage sur un support CPG greffé avec de la désoxyguanosine (Perkin Elmer /Applied Biosystems) , d'un dérivé N,N-diispropyl-β-cyanoethyl-phosphoramidite obtenu à partir de la 5'-O-(4,4'-diméthoxytrityl)-N-4-(6-Trifluoracétamidohexyl)-2'-désoxycytidine préparée par trifluoracétylation de la 5'-O-(4,4'-diméthoxytrityl)-N-4-(6-Aminohexyl)-2'-désoxycytidine [ROGET et al. Nucleic Acids Res., 17, 7643-7650, (1989)].On continue ensuite la synthèse de la séquence ci-dessus, à l'issue de la synthèse sur un synthétiseur d'ADN en mode "trityl-on", l'oligonucléotide est est traité par l'ammoniaque concentrée (16h à 55°C) et purifié par HPLC sur une colonne LiChrospher^{®} RP-18E 250-10 (10µm) (Merck, Allemagne) par un gradient d'acétonitrile dans l'acétate de triéthylammonium 50 mM (tampon A: 5% acétonitrile, tampon B: 50% acétonitrile; débit 5ml/min, gradient de 10% B à 60% B en 20 min, isocratique 60% B pendant 5 min, puis gradient de 60% B à 100% B en 5 min) (Oligonucleotide synthesis : A practical approach. Ed M.J. Gait. IRL Press, Oxford) les fractions correspondant à un pic majoritaire (temps de rétention supérieur à 20 min) sont évaporées. Après évaporation et co-évaporation avec de l'eau, l'oligonucléotide partiellement déprotégé ainsi obtenu est détritylé par l'acide acétique à 80% (température ambiante, 30 min), après évaporation et coévaporation, l'oligonucléotide complètement déprotégé est repris dans 50µl de l'hydrogénocarbonate de triéthylammonium (TEAB) 100mM pH~8 et précipité par 1,5 ml de n-butanol. Après centrifugation le surnageant est éliminé et le précipité séché sous vide est repris par 200 µl d'eau, cette solution mère (oligonucléotide dénommé AH-ODN1) est utilisé pour le marquage au cryptate.

### 2°) Couplage d'une molécule de cryptate [TBP-(Eu3+)] sur un oligodésoxynucléotide fonctionnalisé par un bras aminohexyl (AH-ODN1):

Une partie aliquote de la solution mère de l'oligonucléotide obtenu ci-dessus est diluée par 150µl d'une solution aqueuse d'hydrogénocarbonate de triéthylammonium (TEAB) 0,1 M pH 7,8 et on ajoute 60 µl d'une solution de cryptate [TBP-(Eu3+)] activé (4 mg/ml) soit 171 nmol (environ 4 équivalents). Le cryptate [TBP-(Eu3+)] activé (NHS /DCC) est préparée extemporanément à partir de cryptate d'Europium [(bis-bipy)-(bipy-diacide)]lui-même obtenu à partir du cryptate d'Europium [(bis-bipy)-(bipy-dimethylester)] décrit dans l'exemple 4, section A, de la demande EP 0 321 353.

Après 30 min sous agitation on ajoute 15 µl de TEAB 1M pH 8,5 puis évapore sous vide (speed-vac) jusqu'à un volume de 200 µl, on dépose sur une colonne NAP10 (Pharmacia) équilibrée dans un tampon TEAAc 25 mM pH7 contenant 10% d'acétonitrile, on élue par le même tampon selon le protocole du fabriquant, la fraction exclue est collectée dans un volume de 1 ml et cette fraction est concentrée (speed-vac) jusqu'à un volume de 200 µl.

### 3°) Purification du conjugué formé d'un cryptate rrBP-(Eu3+)] et d'un oligodésoxynucléotide fonctionnalisé par un bras aminohexyl (Conjugué KH-ODN1) :

Le conjugué KH-ODN1 est analysé par FPLC sur une colonne mono-Q (Pharmacia)en utilisant les conditions suivantes (tampon A : sodium acétate 20 mM pH 5 contenant 10% d'acétonitrile. tampon B : acétate de sodium 20 mM pH 5 chlorure de lithium 1M contenant 10% d'acétonitrile. Gradient: 0 à 2 min isocratique 20% B, 2 min à 30 min gradient de 20% B à 60% B, débit 1ml/min).

On injecte ensuite la totalité de la fraction exclue provenant de la colonne NAP10 (200 µl) sur la colonne mono-Q, la fraction correspondant au conjugué K-ODN1 est collectée, concentrée jusqu'à 300µl et déssalée sur une colonne NAP10 équilibrée dans un tampon TEAAc 25 mM pH 7 contenant 10% d'acétonitrile. On élue par le même tampon selon le protocole du fabriquant, la fraction exclue est collectée dans un volume de 1 ml. Cette fraction correspondant au conjugué KH-ODN1 pur est caractérisé par un spectre ultra-violet présentant un maximum à 260 nm (composante ODN) est un épaulement vers 305 nm (composante cryptate). On obtient ainsi un oligonucléotide marqué par le cryptate sur le bras aminohexyl fixé en position 4 de la cytosine proche de l'extrémité 3' [séquence 5'-d(T₁₀ G₄ C^{KAH} G)₃'dénommé K-ODN1].

### 4°) On utilise une solution mère du conjugué K- ODN1 , cette solution mère est diluée dans du tampon PBS contenant 0,1% BSA.

On dépose 100 µl de la solution diluée dans les puits d'une plaque de microtitration. Dans les deux premiers puits, on ajoute 100 µl du tampon et dans les puits suivants on dépose 100 µl d'une dilution d'acide urique préparée de façon à ce que la concentration finale d'acide urique augmente d'un facteur 2 d'un puits à l'autre selon le tableau 4 ci-dessous.

On mesure l'intensité de fluorescence à 620 nm sur un appareil Discovery, on porte les résultats dans le tableau 4 suivant. On calcule le pourcentage d'extinction (quenching) par rapport au témoin ne contenant pas d'acide urique par la relation suivante :
100-100[E₆₂₀ (ac. urique / E₆₂₀ (standard 0)]

**TABLEAU 4**

| [acide urique]finale (mg/l) | E620 (uf) | Extinction (%) |
|---|---|---|
| 0 | 134464 | 0 |
| 1,25 | 128729 | 4, 3 |
| 2,5 | 132572 | 1,4 |
| 5 | 120999 | 10 |
| 10 | 116689 | 13,22 |
| 20 | 111623 | 17,0 |
| 40 | 105703 | 21,4 |
| 80 | 104481 | 22,3 |

On effectue la même série de mesure après des temps d'incubation croissants (indiqués en heures) et de la même façon on mesure l'extinction (%) en fonction de la concentration en acide urique.

Les résultats sont regroupés dans le tableau 5 ci-dessous.

**TABLEAU 5**

| | Extinction (%) | | | |
|---|---|---|---|---|
| [acide urique]finale (mg/l) | t = 0 | t = 3,5 h | t = 5,5 h | t = 16 h |
| 0 | 0 | 0 | 0 | 0 |
| 1,5 | 14 | 13 | 15 | 18 |
| 3,1 | 15 | 17 | 22 | 45 |
| 6,2 | 18 | 19 | 24 | 54 |
| 12,5 | 16 | 26 | 32 | 63 |
| 25 | 25 | 24 | 33 | 72 |
| 50 | 20 | 25 | 34 | 75 |
| 100 | 23 | 34 | 42 | 77 |

Les résultats montrent que pour une concentration donnée d'acide urique (50mg/l) qui provoque une extinction faible de la fluorescence du cryptate lorsqu'il est couplé à l'oligonucléotide (environ 20%), on observe au cours du temps une augmentation de l'extinction jusqu'à environ 75% à un temps correspondant à une digestion complète de l'oligonucléotide.

La digestion de l'oligonucléotide pour donner des nucléosides s'accompagne donc d'une variation des propriétés photophysiques du cryptate et une augmentation de sa sensibilité au quenching.

Ces résultats montrent la possibilité de suivre le déroulement d'une réaction par l'évolution des propriétés photophysiques (comme l'extinction) d'un marqueur fluorescent en présence d'un agent d'extinction introduit dans le milieu réactionnel.

### Exemple 5 : Modifications des propriétés photophysiques d'un conjugué cryptate [trisbipyridine(Eu3+)]-oligonucléotide au cours d'une hybridation

### 1°) Marquage d'un ODN AH-kras12M1 par le cryptate.

On utilise un oligonucléotide de séquence 5'-d(^{AH}C ACG CCA CTA GCT CC)₃' modifié en son extrémité 5' par un bras aminohexyl (AH) synthétisé sur support solide.

En suivant le protocole de marquage de l'exemple 4, on obtient dans ce cas un oligonucléotide marqué par le cryptate sur le bras aminohexyl fixé en position 4 du dérivé de la cytosine situé à l'extrémité 5' [séquence ^{5'}d(C^{KAH} ACG CCA CTA GCT CC)_{3'} dénommée K-ODN2].

### 2°) On synthétise l'oligonucléotide ODN3 de séquence ^{5'}d(GGAGCTAGTGGCGT) _{3'}, complémentaire à la séquence K-ODN2 décrite ci-dessus.

### 3°) Comparaison de la durée de vie d'un conjugué oligonucléotide cryptate sous une forme simple brin et sous une forme double brin :

On place en conditions dénaturantes ( 95 °C, 5 min) une solution contenant des quantité équivalentes de l'oligonucléotide K-ODN2 et son complémentaire dans un tampon phosphate 0,1 M NaCl, puis on favorise l'hybridation en incubant 20 min à 30°C.

On mesure la durée de vie de fluorescence du cryptate dans le cas de l'oligonucléotide simple brin K-ODN2 ainsi que dans le cas de l'oligonucléotide K-ODN2 hybridé sur son complémentaire. Les mesures sont faites dans du tampon phosphate pH 7 contenant 0,1 M NaCl, à 20°C sur un appareil LS50 (Perkin Elmer).

Sur l'appareil LS50, la décroissance d'un signal de fluorescence (mesure en temps résolu) permet à partir des mesures de calculer la durée de vie à partir de la relation linéaire qui relie le logarithme des intensités et le temps. La linéarité de cette relation est estimée par le calcul du coefficient de corrélation (méthode des moindres carrés) à l'aide du logiciel fournit avec l'appareil.

Un coefficient de corrélation supérieur à 0,999 traduit une décroissance monoexponentielle et la présence d'une population de molécules homogènes quant à leur durée de vie, alors qu'un coefficient plus petit dénote généralement la coexistence de deux espèces différentes dans le milieu.

On observe le résultat suivant :

| | t (ms) | coefficient de correlation |
|---|---|---|
| K-ODN2 | 1,282 | 0,9995 |
| K-ODN2 + ODN3 | 0,565 | 0,9992 |

Après 15 min à 60°C, c'est à dire au dessus du Tm (température de fusion caractéristique d'un acide nucléique = la température pour laquelle la moitié des molécules sont sous la forme appariée avec leur séquence complémentaire), on observe les résultats suivants :

| | t (ms) | coefficient de corrélation |
|---|---|---|
| K-ODN2 | 1,043 | 0,9980 |
| K-ODN2 + ODN3 | 0,790 | 0,9956 |

La valeur de Tm pour l'hybridation de K-ODN₂ sur son complémentaire dans les conditions utilisées ici, telle qu'estimée par une formule empirique tenant compte de la longueur de l'oligonucléotide, de la concentration en NaCl et de la proportion en guanine et cytosine (J. Sambrook et al., Molecular Cloning, 2^{nd} édition 1989, Cold Spring Harbor Laboratory Press, § 11.46) est d'environ 30°C.

Après retour aux conditions initiales (favorables à l'hybridation), on observe les résultats suivants :

| | t (ms) | coefficient de correlation |
|---|---|---|
| K-ODN2 | 1,205 | 0,9991 |
| K-ODN2 + ODN3 | 0,533 | 0,9991 |

On observe que, dans le cas de l'oligonucléotide K-ODN2 simple brin, la durée de vie est toujours plus élevée que dans le cas de K-ODN2 sous la forme double brin.

Dans le cas de K-ODN2 sous la forme double brin, la durée de vie augmente avec la température, la durée de vie observée étant la résultante de la valeur proche de 1,20 ms observée pour la forme monobrin et de la valeur proche de 0,5 ms observée pour le double brin (la valeur du coefficient de corrélation indique la présence de deux espèces).

Après un cycle de dénaturation, un retour aux condition favorables à l'hybridation permet d'observer une diminution de la durée de la forme double brin.

### Exemple 6 : Modifications de la sensibilité à l'extinction d'un conjugué cryptate [trisbipyridine(Eu3+)]-oligonucléotide au cours d'une hybridation

On utilise les oligonucléotides K-ODN2 et ODN3 dont les structures et les synthèses sont décrites dans l'exemple 5.

On prépare une solution mère du conjugué K- ODN2. Cette solution mère est diluée dans du tampon PBS contenant 0,1% BSA de façon à obtenir une solution contenant environ 1,5.10⁻⁸ M de cryptate. Parallèlement, on prépare une solution mère du conjugué K- ODN2 hybridé sur son complémentaire (ODN3). Cette solution mère est diluée dans du tampon PBS contenant 0.1% BSA de façon à obtenir une solution contenant également environ 1,5. 10⁻⁸ M de cryptate.

On dépose 100 µl de la solution diluée dans les puits d'une plaque de microtitration, dans les deux premiers puits on ajoute 100µl du tampon et dans les puits suivants on dépose 100 µl d'une dilution d'acide urique dans le tampon PBS, préparée de façon à ce que la concentration finale d'acide urique augmente d'un facteur 2 d'un puits à l'autre selon le tableau 6 ci-dessous.

On mesure l'intensité de la fluorescence à 620 nm des différents puits à température ambiante (20°C) sur un appareil Discovery (mesure en temps résolu avec une fenêtre de 50 à 400 µs). Les résultats sont rapportés dans le tableau 6 suivant et on calcule l'extinction par rapport au témoin ne contenant pas d'acide urique.

**TABLEAU 6**

| Extinction par l'acide urique à 20°C | | | | |
|---|---|---|---|---|
| | K-ODN Simple brin | | K-ODN Double brin | |
| [acide urique]finale (mg/l) | E620 (uf) | Extinction (%) | E620 (uf) | Extinction (%) |
| 0 | 134464 | 0 | 170792 | 0 |
| 1,25 | 128729 | 4,3 | 150359 | 12 |
| 2,5 | 132572 | 1,4 | 122602 | 28,2 |
| 5 | 120999 | 10 | 103991 | 39 |
| 10 | 116689 | 13,22 | 72621 | 57,5 |
| 20 | 111623 | 17 | 57258 | 66,5 |
| 40 | 105703 | 21,4 | 41272 | 75,8 |
| 80 | 104481 | 22,3 | 34824 | 79,6 |

On incube ensuite la plaque pendant 15 min à 60°C et on effectue immédiatement une mesure des intensités de fluorescence à 620nm des différents puits à la température de 60°C sur un appareil Discovery (mesure en temps résolu avec une fenêtre de 50 à 400 µs). Les résultats sont rapportés dans le tableau 7 suivant et on calcule l'extinction par rapport au témoin ne contenant pas d'acide urique.

**TABLEAU 7**

| Extinction par l'acide urique à 60°C | | | | |
|---|---|---|---|---|
| | K-ODN Simple brin | | K-ODN Double brin | |
| [acide urique] finale (mg/l) | E620 (uf) | Extinction (%) | E620 (uf) | Extinction (%) |
| 0 | 65373 | 0 | 71357 | 0 |
| 1,25 | 61383 | 6,1 | 67168 | 5,9 |
| 2,5 | 61048 | 6,6 | 59702 | 16,3 |
| 5 | 57930 | 11,4 | 57534 | 19,4 |
| 10 | 53767 | 17,8 | 57561 | 19,3 |
| 20 | 48357 | 26,0 | 54074 | 24,2 |
| 40 | 46817 | 28,4 | 52864 | 25,9 |
| 80 | 47879 | 26,8 | 50103 | 29,8 |

Ces résultats confirment que la sensibilité du cryptate vis à vis du phénomène d'extinction est différente pour un conjugué cryptate-oligonucléotide lorsque celui ci est sous la forme double brin ou simple brin. L'acide urique est un agent d'extinction spécifique de la forme double brin.

On peut ainsi suivre le phénomène d'hybridation par mesure de la fluorescence en temps résolu en présence d'un composé permettant une extinction sélective de l'espèce hybridée.

### Exemple 7 : Modification des propriétés photophysiques au cours de l'hydrolyse de fonctions esters :

### 1°) Préparation de l'ester de N-Hydroxysuccinimide :

On utilise un ester de N-hydroxysuccinimide qui présente l'avantage de s'hydrolyser spontanément dans l'eau à un pH 7 avec une vitesse suffisamment lente pour faciliter les mesures de fluorescence et permettre de faire un suivi cinétique des propriétés photophysiques de façon simple.

On utilise un dérivé cryptate-[trisbipyridine(Eu³⁺)]-bis-aminoéthylcarboxamide (produit dénommé KNH2 dans l'exemple 1) obtenu par aminolyse en présence d'éthylènediamine du cryptate d'Europium [(bis-bipy)-(bipy-diéthylester)] décrit dans l'exemple 4, section A, de la demande EP 0 321 353 (Mathis G. et Lehn J.M.).

On fait réagir ce cryptate sur un excès de DSS (ester bis(N-hydroxysuccinimide de l'acide subérique, Sigma).

Le dérivé KNH2 caractérisé en HPLC par un Rt de 12,3 min est transformé en ester de NHS [K-di(NHS-Suberate)] montrant un Rt de 17,2 min [conditions HPLC analytique colonne Lichrospher (Merck) RP-18 (5 µ) 125-4 élution à 1 ml/mn Solvant A : 1 % TFA dans H₂O; solvant B : Acétonitrile pur. Gradient de 15 % B à 32 % B en 30 min].

Le cryptate ainsi obtenu portant deux fonctions ester [K-di(NHS-Suberate)] est purifié par HPLC préparative sur une colonne Lichrospher (Merck) RP-18 (10 µ) 250-10 élution à 5 ml/mn [Solvant A : 1 % TFA dans H₂O : solvant B : Acétonitrile pur. Gradient de 15 % B à 32 % B en 30 min]. La fraction contenant l'ester pur est évaporée sous vide puis séché, une analyse HPLC (conditions analytiques ci-dessus) montre un temps de rétention de 17,2 min.

De façon analogue, on prépare l'ester de NHS [K-mono-NHS-Suberate)], en partant d'un dérivé cryptate-[trisbipyridine(Eu³⁺)]mono-aminoéthylcarboxamide obtenu par aminolyse en présence d(éthylènediamine du cryptate d'Europium [(bis-bipy)-(bipy-monoéthylester)] lui même préparé par saponification partielle (NaOH aqueux puis purification RP-HPLC) du cryptate d'Europium [(bis-bipy)-(bipy-diéthylester)] décrit dans l'exemple 4, section A, de la demande EP 0 321 353.

Cet ester est purifié par RP-HPLC préparative (conditions ci-dessus), la fraction contenant le mono-ester pur (Rt=15,6 min) est rapidement évaporée sous vide. Ce monoester sera utilisé pour l'étude de la modification des propriétés de fluorescence au cours de l'hydrolyse.

Par analyse HPLC dans le gradient ci-dessus, on observe qu'en solution aqueuse ou en solution tamponnée pH 7, le diester se convertit en dérivé monoacide de Rt 16,0 min puis en dérivé diacide de Rt 14,8 min. Dans l'eau, on observe une hydrolyse de plus de 50 % en 3 h.

Par analyse HPLC dans le gradient ci-dessus, on observe qu'en solution aqueuse ou en solution tamponnée pH7, le mono-ester de Rt 15,6 se convertit en dérivé acide de Rt 14,2 mn. Dans l'eau, on observe une hydrolyse de plus de 50 % en 3 h.

### 2°) Modification des spectres de fluorescence au cours de l'hydrolyse d'un ester de N-hydroxysuccinimide de cryptate-[trisbipyridine(Eu³⁺)] :

On enregistre les spectres d'émission de fluorescence et la durée de vie de fluorescence de cryptate K-NHS-Suberate en solution aqueuse, en effectuant une mesure juste après la dilution puis après 20 h. Dans les mêmes conditions, on enregistre les spectres d'une solution de KNH2 servant de référence (solution de concentration équivalente). On vérifie que l'intensité de la fluorescence émise à 620 nm de cette référence ne varie pas de plus de 2 % en 20 h et que la durée de vie n'est pas modifiée (variation < 0,3 %). Les résultats sont reportés dans le tableau 7 suivant :

**TABLEAU 7**

| temps (h) | E 620 (uf) | Variation E 620 (%) | Fluorescence Totale (Aire) | Variation Fluorescence Totale(%) | Durée de vie (µs) | Coefficient de Corrélation |
|---|---|---|---|---|---|---|
| 0 | 81,8 | - | 1475 | - | 376 | 0,9999 |
| 20 | 96,0 | + 17,6 | 2198 | + 49 % | 458 | 0.9999 |

On observe qu'au cours de l'hydrolyse de la fonction ester les propriétés photophysiques tel que l'intensité d'émission ou la durée de vie de fluorescence sont modifiées.

## Revendications

1. Méthode homogène de détection et/ou de détermination par fluorescence d'une interaction chimique ou physicochimique dans un milieu de mesure, dans laquelle on utilise un cryptate de terre rare comportant un substituant, **caractérisée en ce que** le cryptate de terre rare est le seul traceur fluorescent utilisé et **en ce qu'**on mesure la variation d'au moins une caractéristique de fluorescence du cryptate de terre rare induite par la modification des propriétés physicochimiques du substituant résultant de ladite interaction, ledit substituant étant un groupe phosphate, phosphonate , carboxylate ou un de leurs dérivés, ou une molécule chimique ou biologique comportant ce groupe, ladite molécule étant un acide nucléique, un oligonucléotide ou un oligonucléotide constitué de ou comprenant des analogues de nucléotides; un nucléotide ou un nucléoside et leurs analogues, sous réserve que ladite interaction ne soit pas une réaction enzymatique catalysée par la terminale nucléotidyl transférase, lorsque le cryptate de terre rare est le cryptate d'europium trisbipyridine et la caractéristique de fluorescence est la durée de vie.

2. Méthode selon la revendication 1, **caractérisée en ce que** ladite modification influe sur la sphère de solvatation de l'ion terre rare.

3. Méthode selon les revendications 1 ou 2, **caractérisée en ce que** la caractéristique de fluorescence est choisie parmi la durée de vie d'émission, l'intensité de fluorescence, la répartition des raies dans le spectre de fluorescence et la polarisation.

4. Méthode selon l'une des revendications 1 à 3, **caractérisée en ce que** la variation est une augmentation ou une diminution de la durée de vie d'émission ou de l'intensité de fluorescence.

5. Méthode selon l'une quelconque des revendications 1 à 4, **caractérisée en ce que** ladite interaction résulte d'une réaction enzymatique.

6. Méthode selon l'une quelconque des revendications 1 à 5, **caractérisée en ce que** ladite interaction consiste en une réaction d'hybridation ou de dénaturation d'acides nucléiques, d'oligonucléotides ou d'oligonucléotides constitués de ou comprenant des analogues de nucléotides ou en une réaction entre lesdits acides nucléiques ou oligonucléotides avec des protéines ayant une affinité pour ceux-ci.

7. Méthode selon l'une quelconque des revendications 1 à 6, **caractérisée en ce que** le cryptate de terre rare est lié au substituant de manière covalente, soit directement, soit par l'intermédiaire d'un bras d'espacement.

8. Méthode selon l'une quelconque des revendications 1 à 7, **caractérisée en ce que** ledit cryptate de terre rare est constitué d'au moins un sel de terre rare complexé par un composé macropolycyclique de formule 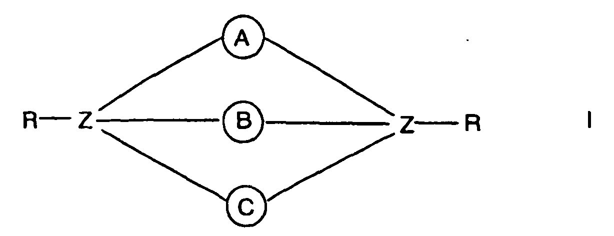 dans laquelle Z est un atome ayant 3 ou 4 valences, R est rien ou représente l'hydrogène, le groupe hydroxy, un groupe amino ou un radical hydrocarboné, les radicaux bivalents Ⓐ, Ⓑ et Ⓒ, sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ, Ⓑ et Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

9. Méthode selon la revendication 8, **caractérisée en ce que** le cryptate de terre rare est constitué d'un sel de terre rare complexé par l'un des composés macrocycliques ou macropolycycliques ci-après :
(22) phénanthroline ; (22)phénanthroline amide ; (22)anthracène ; (22)anthracène amide ; (22)bi-isoquinoléine ; (22)biphényl-bis-pyridine ; (22) bipyridine ; (22) bi-pyridine amide; les macropolycycles tris-bipyridine, tris-phénanthroline, phénanthroline-bis-bipyridine, bi-isoquinoléine-bis-bipyridine, bis-bipyridine diphénylbipyridine ; un composé macropolycyclique comprenant un motif moléculaire choisi parmi les bipyrazines, les bipyrimidines et les hétérocycles azotés comportant des groupements N-oxydes.

10. Méthode selon l'une quelconque des revendications 1 à 7, **caractérisée en ce que** le cryptate de terre rare est constitué d'au moins un sel de terre rare complexé par un composé macropolycyclique répondant à l'une des formules II ou III ci-après : 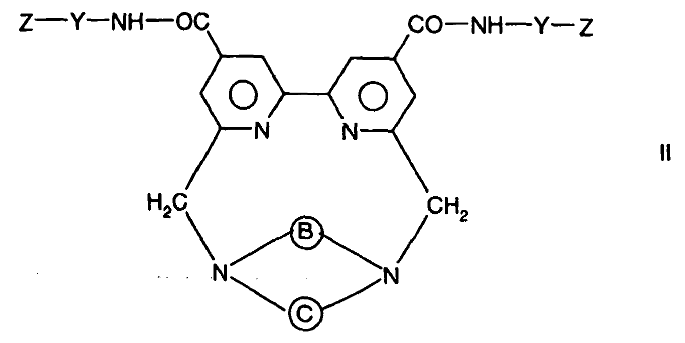 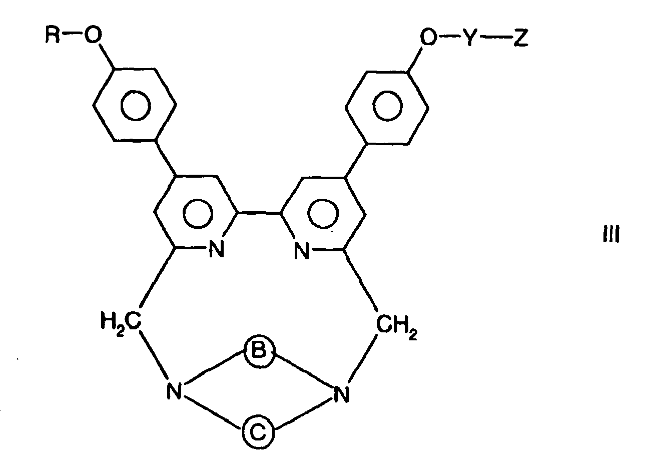 dans lesquels :
- le cycle de formule 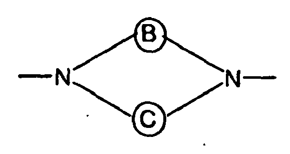 est l'un des cycles suivants :
1) 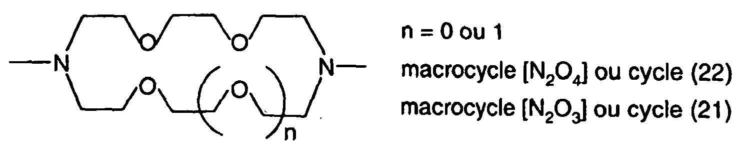
2) 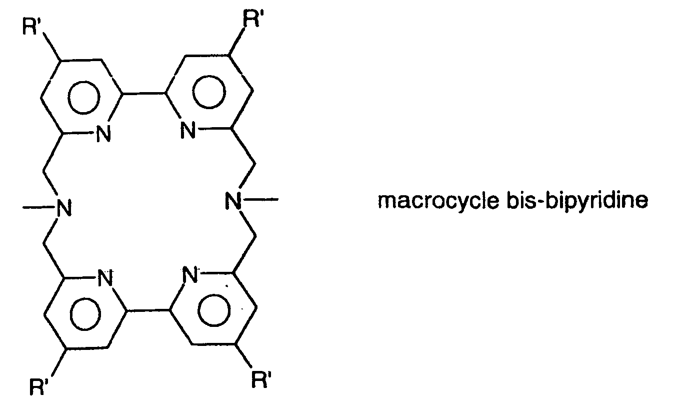
- Y est un groupe ou un bras d'espacement qui est constitué par un radical organique bivalent, choisi parmi les groupes alkylène linéaires ou ramifiés en C₁ à C₂₀ contenant éventuellement une ou plusieurs doubles liaisons et/ou éventuellement contenant un ou plusieurs hétéroatomes tels que l'oxygène, l'azote, le soufre, le phosphore ou un ou plusieurs groupe(s) carbamoyle ou carboxamido ; parmi les groupes cycloalkylène en C₅ à C₈ ou parmi les groupes arylène en C₆ à C₁₄, lesdits groupes alkylène, cycloalkylène ou arylène étant éventuellement substitués par des groupes, alkyle, aryle ou sulfonate ;
- Z est un groupe fonctionnel susceptible de se lier de façon covalente avec une substance biologique ;
- R est un groupe méthyle ou représente le groupe -Y-Z ;
- R' est l'hydrogène ou un groupe -COOR" dans lequel R" est un groupe alkyle en C₁ à C₁₀ et représente de préférence le groupe méthyle, éthyle ou tertiobutyle ou bien R' est un groupe -CO-NH-Y-Z.

11. Méthode selon l'une quelconque des revendications 1 à 10, **caractérisée en ce que** le cryptate de terre rare est un cryptate d'europium, de terbium, de samarium, de dysprosium ou de néodymium.

12. Méthode selon la revendication 11, **caractérisée en ce que** le cryptate de terre rare est le cryptate d'europium Eu trisbipyridine ou Eu[bis-diéthoxy bipyridine.bipyridine].

## Claims

1. A homogeneous method for detecting and/or determining by fluorescence a chemical or physicochemical interaction in a measuring medium, in which a rare-earth metal cryptate comprising a substituent is used, **characterised in that** the rare-earth metal cryptate is the only fluorescent tracer used and that the variation of at least one fluorescence characteristic of the rare-earth metal cryptate, induced by the change in the physicochemical properties of the substituent resulting from said interaction, is measured, the said substituent being a group of phosphate, phosphonate, carboxylate, or a product derived thereof, or a chemical or biological molecule comprising the group, the said molecule being a nucleic acid, an oligonucleotide or an oligonucleotide consisting of or comprising nucleotide analogs; a nucleotide or a nucleoside and their analogs, provided that the said interaction is not an enzymatic reaction catalysed by the terminal nucleotidyl transferase, when the rare-earth metal cryptate is europium trisbipyridine cryptate and the fluorescence characteristic is the emission lifetime.

2. The method according to claim 1, **characterised in that** said change has an influence on the solvation sphere of the rare-earth metal ion.

3. The method according to claim 1 or 2, **characterised in that** the fluorescence characteristic is chosen from the emission lifetime, the fluorescence intensity, the distribution of the lines in the fluorescence spectrum and the polarisation.

4. The method according to one of claims 1 to 3, **characterised in that** the variation is an increase or decrease in the emission lifetime or the fluorescence intensity.

5. The method according to any one of claims 1 to 4, **characterised in that** the said interaction results from an enzymatic reaction.

6. The method according to any one of the claims 1 to 5, **characterised in that** the said interaction consist of a hybridisation reaction or denaturing reaction of nucleic acids, oligonucleotides or oligonucleotides consisting of or comprising nucleotide analogs or of a reaction between the said nucleic acids or oligonucleotides with proteins having any affinity for them.

7. The method according to any one of claims 1 to 6, **characterised in that** the rare-earth metal cryptate is linked covalently to the substituent, either directly or via a spacer arm.

8. The method according to any one of claims 1 to 7, **characterised in that** said rare-earth metal cryptate consists of at least one salt of a rare-earth metal complexed with a macropolycyclic compound of formula 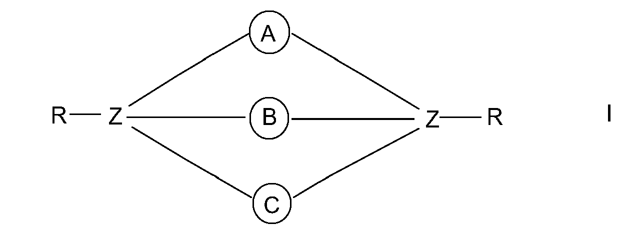 in which Z is an atom with 3 or 4 valences, R is nothing or represents hydrogen, a hydroxyl group, an amino group or a hydrocarbon-based radical, the divalent radicals Ⓐ, Ⓑ and Ⓒ are, independently of each other, hydrocarbon-based chains optionally containing one or more hetero atoms and optionally interrupted with a heteromacrocycle, at least one of the radicals Ⓐ, Ⓑ and Ⓒ, also comprising at least on molecular unit or consisting essentially of a molecular unit, said molecular unit having a triplet energy greater than that of the emission level of the complexed rare-earth metal ion.

9. The method according to claim 8, **characterised in that** the rare-earth metal cryptate consists of a salt of a rare-earth metal complexed with one of the macrocyclic or macropolycyclic compounds below:
(22)phenanthroline; (22)phenanthroline amide; (22)anthracene, (22)anthracene amide, (22)bi-isoquinoline; (22)biphenyl-bis-pyridine; (22)bipyridine; (22)bi-pyridine amide; tris-bipyridine macropolycycles, tris-phenanthroline; phenanthroline-bis-bipyridine; bi-isoquinoline-bis-bipyridine; bis-bipyridine diphenylbipyridine; a macropolycyclic compound comprising a molecular unit chosen from bipyrazines; bipyrimidines and nitrogenous heterocycles comprising N-oxide groups.

10. The method according to claims 1 to 7, **characterised in that** the rare-earth metal cryptate consists of at least one salt of a rare-earth metal complexed with a macropolycyclic compound corresponding to one of the formulae II and III below: 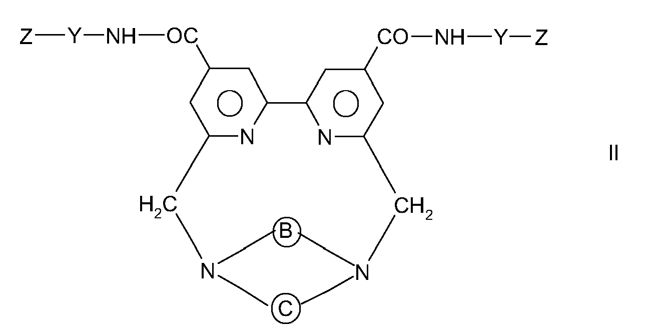 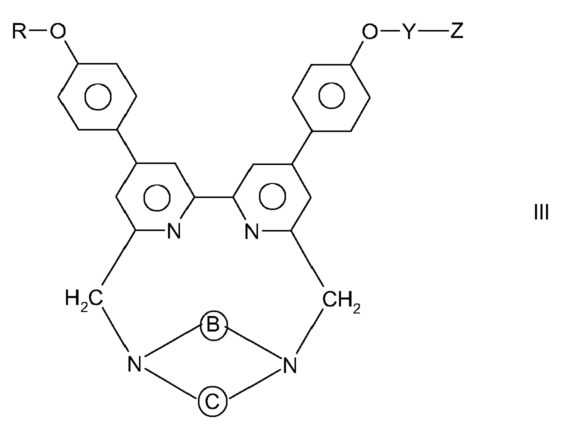 in which:
the ring of formula 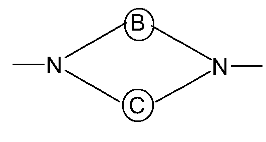
is one of the following rings:
1) 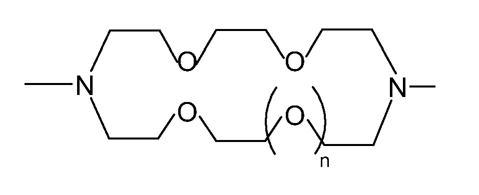
2) 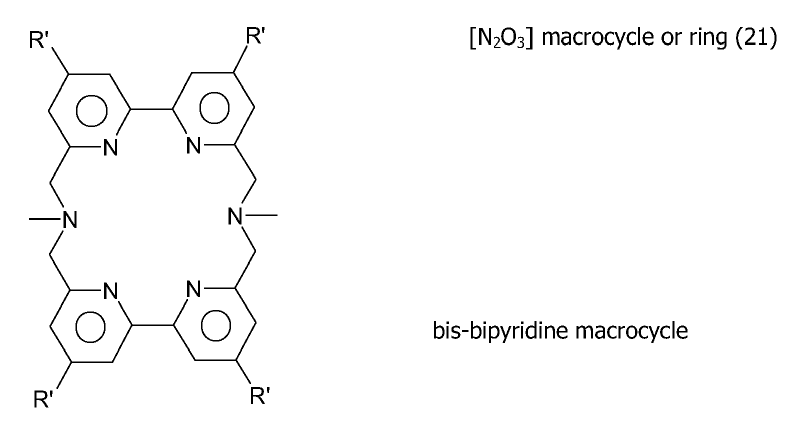 n=0 or 1
[N₂O₄] macrocycle or ring (22)
Y is a group or a spacer arm which consists of a divalent organic radical, chosen from linear or branched C₁ to C₂₀ alkylene groups optionally containing one or more double bonds and/or optionally containing one or more hetero atoms such as oxygen, nitrogen, sulphur or phosphorus or one or more carbamoyl or carboxamido groups; from C₅ to C₈ cycloalkylene groups or from C₆ to C₁₄ arylene groups, said alkylene, cycloalkylene or arylene groups optionally being substituted with alkyl, aryl, or sulfonate groups;
Z is a functional group capable of bonding covalently with a biological substance;
R is a methyl group or represents a group -Y-Z;
R' is hydrogen or a group -COOR" in which R" is a C₁ to C₁₀ alkyl group and preferably represents a methyl, ethyl or tert-butyl group or R' is a group -CO-NH-Y-Z.

11. The method according to claims 1 to 10, **characterised in that** the rare-earth metal cryptate is a europium, terbium, samarium, dysprosium or neodymium cryptate.

12. The method according to claim 11, **characterised in that** the rare-earth metal cryptate is the europium cryptate Eu tris-bipyridine or Eu[bis-diethoxybipyridine.bipyridine].

## Patentansprüche

1. Homogenes Verfahren zur Detektion und/oder Bestimmung durch Fluoreszenz von einer chemischen oder physikochemischen Wechselwirkung in einem Meßmedium, in welchem man ein Seltenerdcryptat verwendet, daß einen Substituenten aufweist, **dadurch gekennzeichnet, daß** das Seltenerdcryptat der einzige verwendete Fluoreszenz-Tracer ist und dadurch, daß man die Variation eines Fluoreszenzmerkmals des Seltenerdcryptats mißt, das durch die Modifikation der physikochemischen Eigenschaften des Substituenten resultierend aus der Wechselwirkung induziert ist, wobei der Substituent eine Phosphat-, Phosphonat-, Carboxylatgruppe oder eines von ihren Derivaten ist oder ein chemisches oder ein biologisches Molekül, das diese Gruppe umfaßt, wobei das Molekül eine Nukleinsäure, ein Oligonukleotid oder ein Oligonukleotid ist, das aus Nukleotidanaloga besteht oder diese umfaßt, ein Nukleotid oder ein Nukleosid und deren Analoga mit der Bedingung, daß die Wechselwirkung keine Enzymreaktion ist, die katalysiert wird durch die terminale Nukleotidyltranferase, wenn das Seltenerdcryptat Europium-Tris-Bipyridincryptat ist und das Fluoreszenzmerkmal die Lebensdauer ist.

2. Verfahren nach Anspruch 1, **dadurch gekennzeichnet, daß** die Modifikation die Solvatationsumgebung des Seltenerdions beeinflußt.

3. Verfahren nach einem der Ansprüche 1 oder 2, **dadurch gekennzeichnet, daß** das Fluoreszenzmerkmal gewählt ist unter der Emissionslebensdauer, der Fluoreszenzintensität, der Verteilung der Strahlen im Fluoreszenz- und Polarisationsspektrum.

4. Verfahren nach einem der Ansprüche 1 bis 3, **dadurch gekennzeichnet, daß** die Variation eine Vergrößerung oder Verminderung der Emissionslebensdauer oder der Fluoreszenzintensität ist.

5. Verfahren nach einem der Ansprüche 1 bis 4, **dadurch gekennzeichnet, daß** die Wechselwirkung aus einer enzymatischen Reaktion resultiert.

6. Verfahren nach einem der Ansprüche 1 bis 5, **dadurch gekennzeichnet, daß** die Wechselwirkung in einer Hybridisierungs- oder Denaturierungsreaktion von Nuleotidensäuren, Oligonukleotiden oder Oligonuleotiden besteht, die aus Nukleotidanaloga bestehen oder diese umfassen, oder in einer Reaktion unter den Nukleinsäuren oder Oligonukleotiden mit Proteinen mit einer Affinität zu diesen besteht.

7. Verfahren nach einem Ansprüche 1 bis 6, **dadurch gekennzeichnet, daß** das Seltenerdcryptat kovalent an den Substituenten entweder direkt oder mittels eines Beabstandungsarmes gebunden ist.

8. Verfahren nach einem der Ansprüche 1 bis 7, **dadurch gekennzeichnet, daß** das Seltenerdcryptat aus wenigstens einem Seltenerdsalz besteht, das komplexiert ist durch eine makropolyzyklische Verbindung mit einer Formel 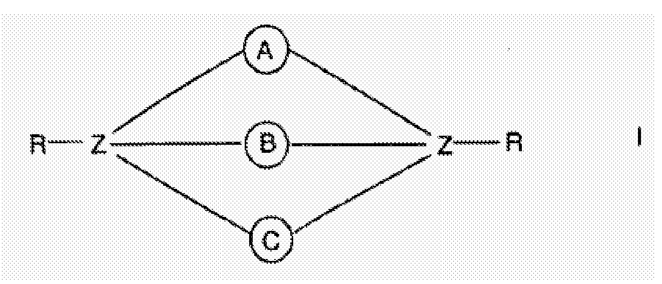 in welcher Z ein Atom mit drei oder vier Valenzen ist, R nichts ist oder Wasserstoff repräsentiert, die Hydroxygruppe, eine Aminogruppe oder einen Kohlenwasserstoffrest darstellt, die bivalenten Reste Ⓐ, Ⓑ und Ⓒ unabhängig voneinander Kohlenwasserstoffketten sind, die gegebenenfalls eines oder mehrere Heteroatome enthalten und gegebenenfalls unterbrochen sind durch einen Heteroaromatenring mit wenigstens einem der Reste Ⓐ, Ⓑ und Ⓒ, umfassend wenigstens ein Molekülmotiv oder im wesentlichen bestehend aus einem Molekülmotiv, wobei das Molekülmotiv eine Triplettenergie über jener des Emissionsniveaus des komplexierten Seltenerdions aufweist.

9. Verfahren nach Anspruch 8, **dadurch gekennzeichnet, daß** das Seltenerdcryptat besteht aus einem Seltenerdsalz, das komplexiert ist durch eine der nachfolgend genannten makrozyklischen oder makropolyzyklischen Verbindungen: (22)Phenanthrolin; (22)Phenanthrolinamid; (22)Anthracen; (22)Anthracenamid; (22)Biisoquinolein; (22)Bipheynyl-Bis-Pyridin; (22)Bipyridin; (22)Bipyridinamid; die Makropolyzyklen Tris-Bipyridin, Tris-Phenanthrolin, Phenanthrolin-Bis-Bipyridin, Biisoquinolein-Bis-Bipyridin, Bis-Bipyridin-Diphenylbipyridin; einer makropolyzyklischen Verbindung, die ein Molekülmotiv umfasst, das gewählt unter Bipyrazinen, Bipyrimidinen und den stickstoffhaltigen Heterozyklen, umfassend die N-Oxydgruppen.

10. Verfahren nach einem der Ansprüche 1 bis 7, **dadurch gekennzeichnet, daß** das Seltenerdcryptat besteht aus wenigstens aus einem Seltenerdsalz, das komplexiert ist durch eine makropolyzyklische Verbindung, die einer der nachfolgenden Formeln II oder III entspricht: 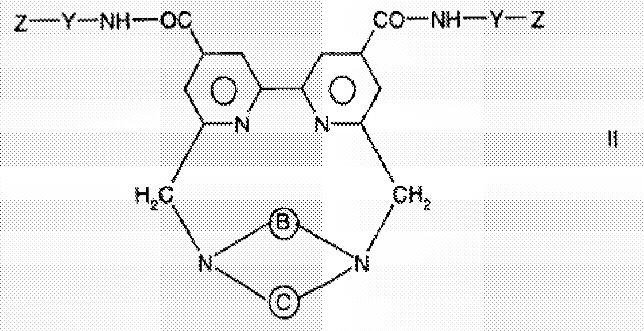 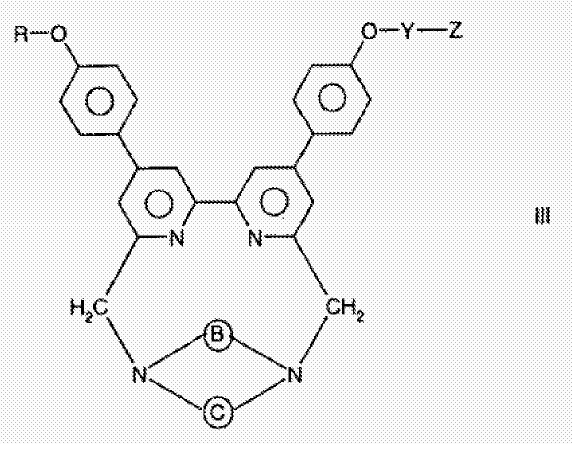 in denen:
- der Ring einer Formel 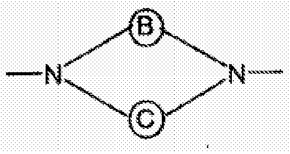
1) 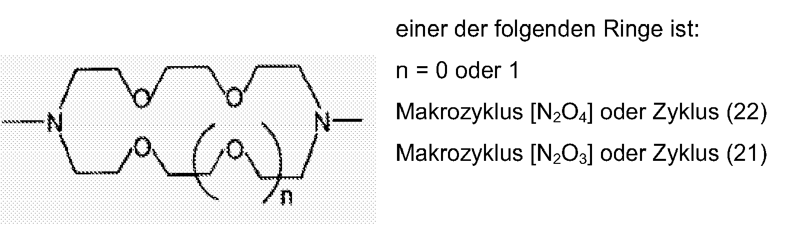
2) 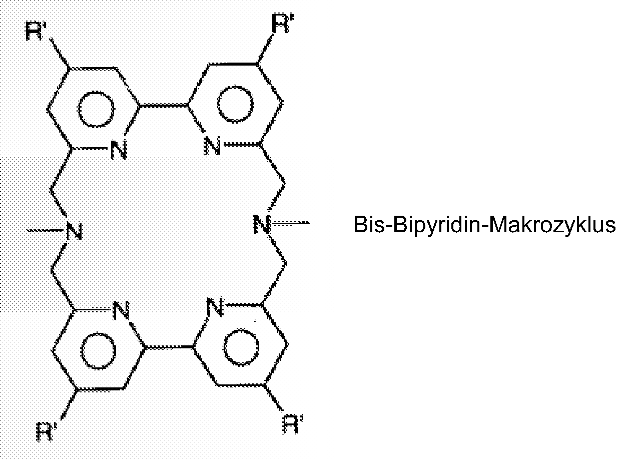
- Y eine Gruppe oder ein Beabstandungsarm ist, welche (welcher) besteht aus einem organischem, bivalenten Rest, gewählt unter den linearen oder verzweigten C₁- bis C₂₀-Alkylengruppen, der gegebenenfalls eine oder mehrere Doppelbindungen enthält und/oder gegebenenfalls eins oder mehrere Heteroatome enthält wie Sauerstoff, Stickstoff, Schwefel, Phosphor oder eine oder mehrere Carbamoyl- oder Carboxamidgruppen, unter den C₅- bis C₈-Zykloalkylengruppen oder unter den C₆- bis C₁₄- Arylengruppen, wobei die Alkylen-, Cykloalkylen- oder Arylengruppen gegebenenfalls substituiert sind durch Alkyl-, Aryl- oder Sulfonatgruppen;
- Z eine funktionelle Gruppe ist, die geeignet ist, sich kovalent mit einer biologischen Substanz zu verbinden;
- R eine Methylgruppe ist oder die Gruppe -Y-Z darstellt;
- R' Wasserstoff oder eine -COOR"-Gruppe ist, in der R" eine C₁- bis C₁₀-Alkylgruppe ist und vorzugsweise die Methyl-, Ethyl- oder tert.-Butylgruppe darstellt oder auch R' eine Gruppe -CO-NH-Y-Z ist.

11. Verfahren nach einem der Ansprüche 1 bis 10, **dadurch gekennzeichnet, daß** das Seltenerdcryptat ein Europium-, Terbium-, Samarium-, Dysprosium- oder Neodymcryptat ist.

12. Verfahren nach Anspruch 11, **dadurch gekennzeichnet, daß** das Seltenerdcryptat das Europiumcryptat Eu-Tris-Pyridin oder Eu[Bis-Diethoxy-Bipyridin.Bipyridin] ist.
